# EUROPEAN PATENT APPLICATION

(11) **EP 0 646 792 A1**
(43) Date of publication of application: **05.04.1995**
(21) Application number: 94306053.3
(22) Date of filing: 17.08.1994
(51) Int. Cl.: G01N 33/50, G01N 33/68, C07K 14/47, C07K 14/575, C07K 14/81

(54) **Use of amylin in pharmaceutical screens**

(30) Priority: 19.08.1993 US 109782
(71) Applicant: ELI LILLY AND COMPANY, Indianapolis, Indiana 46285 (US); ATHENA NEUROSCIENCES, INC., South San Francisco, CA 94080 (US)
(72) Inventor: May, Patrick Cornelious, Carmel, Indiana 46032 (US); Rydel, Russell Eugene, Belmont, California 94002 (US)
(74) Representative: Tapping, Kenneth George

(57) **Abstract**

This invention describes a series of assays useful in evaluating the efficacy of agents which inhibit the neurotoxic effects of β-amyloid peptide. These assays employ amyloidogenic amylin or β₂-microglobulin as the target protein instead of the β-amyloid peptide.

## Description

Alzheimer's disease is a degenerative disorder of the human brain. Clinically, it appears as a progressive dementia. Its histopathology is characterized by degeneration of neurons, gliosis, and the abnormal deposition of proteins in the brain. Proteinaceous deposits (called "amyloid") appear as neurofibrillary tangles, amyloid plaque cores, and amyloid of the congophilic angiopathy. [For a review, see, D.J. Selkoe, Neuron, 6:487-498 (1991)]

While there is no general agreement as to the chemical nature of neurofibrillary tangles, the major constituent of both the amyloid plaque cores and the amyloid of the congophilic angiopathy has been shown to be a 4500 Dalton protein originally termed β-protein or amyloid A4. Throughout this document this protein is referred to as β-amyloid peptide or protein.

β-amyloid peptide is proteolytically derived from a transmembrane protein, the amyloid precursor protein. Different splice forms of the amyloid precursor protein are encoded by a widely expressed gene. see. e.g., K. Beyreuther and B. Muller-Hill, Annual Reviews in Biochemistry, 58:287-307 (1989). β-amyloid peptide consists, in its longest forms, of 42 or 43 amino acid residues. J. Kang, et al., Nature (London), 325:733-736 (1987). These peptides, however, vary as to their amino-termini. C. Hilbich, et al., Journal of Molecular Biology, 218:149-163 (1991).

Because senile plaques are invariably surrounded by dystrophic neurites, it was proposed early that β-amyloid peptide is involved in the loss of neuronal cells that occurs in Alzheimer's disease. B. Yankner and co-workers were the first to demonstrate that synthetic β-amyloid peptide could be neurotoxic in vitro and in vivo. B.A. Yankner, et al., Science, 245:417 (1989); See, also, N.W. Kowall, et al., Proceedings of the National Academy of Sciences. U.S.A., 88:7247 (1991). Other research groups, however, were unable to consistently demonstrate direct toxicity with β-amyloid peptide. See, e.g., Neurobiology of Aging, 13:535 (K. Kosik and P. Coleman, eds. 1992). Even groups receiving β-amyloid peptide from a common source demonstrate conflicting results. P. May, et al., Neurobiology of Aging, 13:605-607 (1991).

In addition to Alzheimer's disease, Down's syndrome is also characterized by an accumulation of β-amyloid peptide. In patients suffering from Down's syndrome the β-amyloid peptide is the primary constituent of senile plaques and cerebrovascular deposits.

Because of the debilitating effects of Alzheimer's disease and Down's syndrome there continues to exist a need for effective treatments. This invention provides assay systems which are useful to evaluate the efficacy of potential agents to treat this disease.

Human amylin (also known as diabetes-associated peptide and islet amyloid polypeptide) is a 37 amino acid peptide first isolated from amyloid deposits in the pancreas of non-insulin-dependent diabetic individuals. C.J.S. Cooper et al., Proceedings of the National Academy of Sciencs (USA), 84:8628-8632 (1987). The rat homologue of amylin is 95% similar in primary sequence to human amylin but has amino acid substitutions in the region conferring β-pleated secondary structure which render it non-amyloidogenic. See. e.g., J. Asai et al., Biochemical and Biophysical Research Communications, 164:400-405 (1989); L.R. McLean and A. Balasubramaniam, Biochimica and Biophysica Acta, 112:317-320(1992).

β₂-microglobulin is another protein unrelated to β-amyloid peptide but capable of forming amyloid deposits in the kidney. [for recent review, see, J.M. Campistol and M. Skinner, Seminars in Dialvsis, **6**:117-126 (1993)]. This nonpolymorphic peptide is homologous to the C3 domain of the immunoglobulin class IgG and is one subunit of class I major histocompatibility antigens.

The inconsistency of existing β-amyloid neurotoxicity assays makes the availability of more sensitive assays having a broader assay window of benefit in the search for agents effective against conditions associated with an excess of β-amyloid peptide-induced neurotoxicity.

This invention describes methods for assessing the effectiveness of an agent useful for ameliorating the neurotoxic effects associated with accumulation of β-amyloid peptides which comprises
a. incubating potential inhibitors of neurotoxicity with an amyloidogenic amylin or β₂-microglobulin;
b. measuring the neurotoxic properties of each amyloidogenic amylin or β₂-microglobulin/potential inhibitor mixture; and
c. detecting reduction in the neurotoxicity relative to a control.

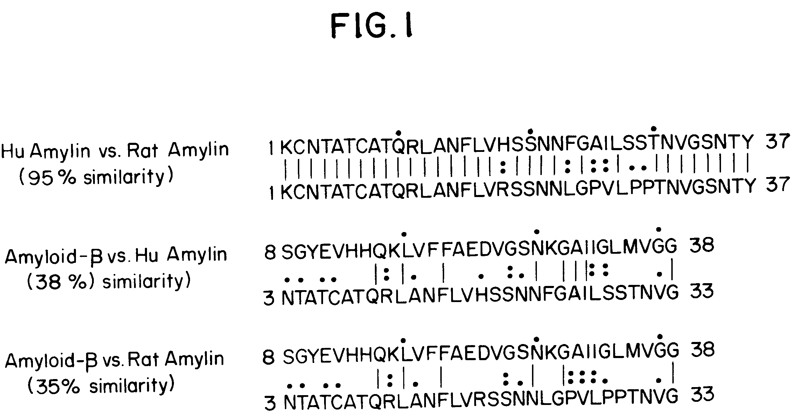

Figure 1 depicts the amino acid sequence similarity between amylin and amyloid-β. Peptide sequences were translated from cDNA entries in GenBank® and the percent similarity identified using the BestFit® program. Top sequence in each pair represents the first peptide mentioned in the comparison. Lines indicate identity, colons represent conservative substitutions while non-similarities are identified by periods or gaps.

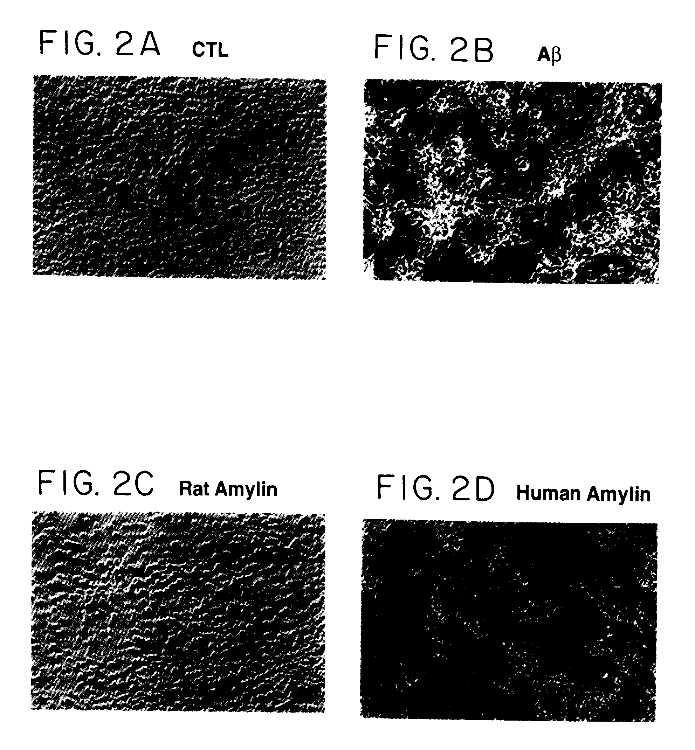

Figure 2 depicts neurodegeneration induced by amyloidogenic peptides. Photomicrographs of rat hippocampal cultures [10 DIV (days in vitro)] treated for 4 days under the following conditions: (**A**) Vehicle control (H₂O), (**B**) 50 µM β-amyloid peptide, (**C**) 50 µM rat amylin, (**D**) 50 µM human amylin. (magnification at 200 X)

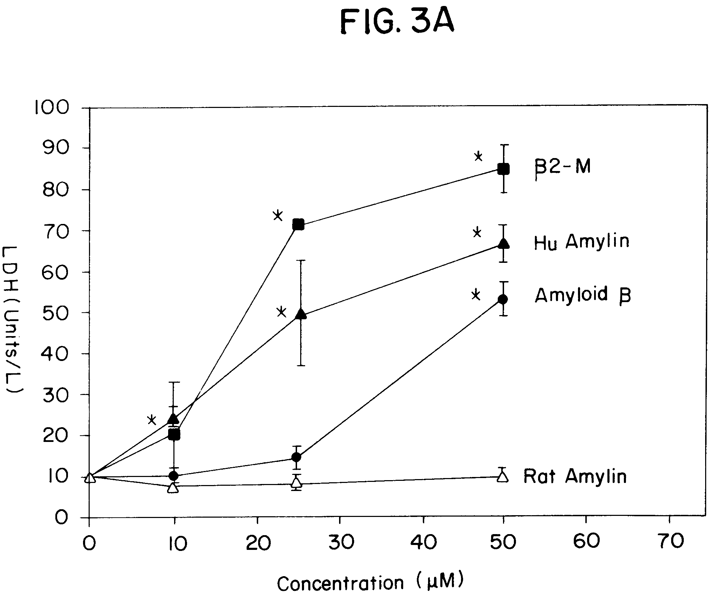

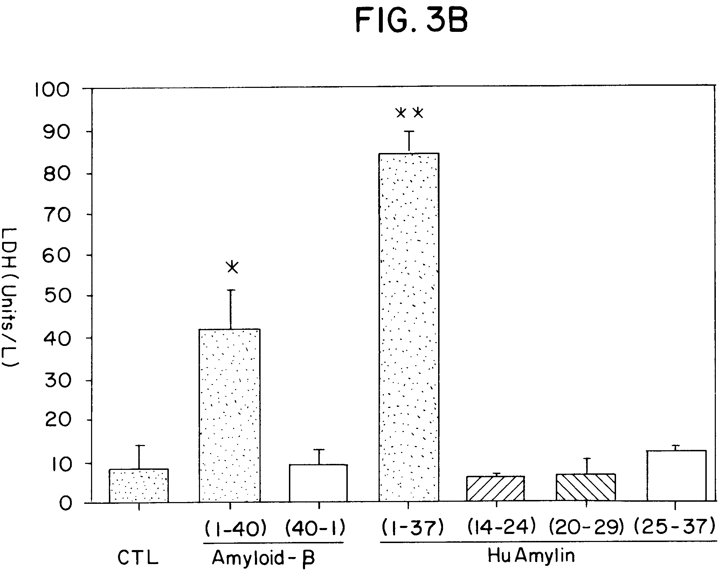

Figure 3, panel A, shows the concentration-dependent neurotoxicity of amyloidogenic peptides. Peptides or vehicle (H₂O) were added at indicated concentrations to mature (10 DIV) rat hippocampal cultures and viability assessed 4 days later. Results are expressed as units/L of LDH (mean ± SEM, n=3) and are representative of 3-5 separate experiments. * p < 0.01 for difference from control.

Figure 3, panel B, depicts the toxicity of human amylin fragments. Mature rat hippocampal cultures were treated for 4 days with 50 µM human amylin (1-37) or various human amylin fragments. Sister cultures were treated with an equivalent concentration of β-amyloid peptide(1-40) or reverse β-amyloid peptide(40-1). Results are expressed as units/L of lactate dehydrogenase (LDH) (mean ± SD, n=3) and are representative of 2-5 separate experiments. *p < 0.01 for difference from control; ** p< 0.01 for difference from β-amyloid peptide(1-40).

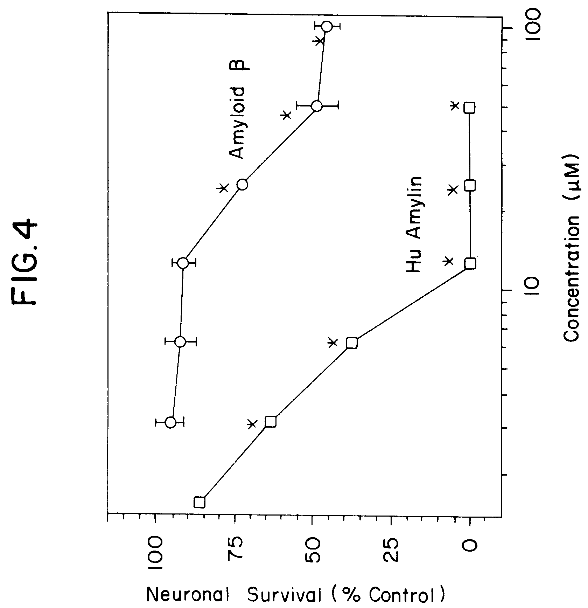

Figure 4 shows the concentration-dependent neurotoxicity of amyloidogenic peptides in human cortical cultures. Peptides or vehicle (H₂O) were added at indicated concentrations to mature (19 DIV) human cortical cultures and viability assessed three days later. Results are expressed as percent of vehicle treated (control) cultures (mean ± SD, n=3) and were determined using the flurometric metabolic indicator alamarBlue™.
*p<0.001 for difference from control.

The terms and abbreviations used in the instant examples have their normal meanings unless otherwise designated. For example "°C" refers to degrees Celsius; "N" refers to normal or normality; "mmole" refers to millimole or millimoles; "g" refers to gram or grams; "ml" means milliliter or milliliters; and "M" refers to molar or molarity.

The term "amyloidogenic" as used herein refers to the ability of a peptide to self associate into higher ordered aggregates and eventually assemble into amyloid fibrils.

The term "conservative variant" as used herein refers to amino acid substitutions which alter the primary sequence of the peptide but do not alter its secondary structure or diminish its amyloidogenic properties.

The terms "human amylin(20-29)", "β-amyloid(1-43)" and the like refer to amino acids 20-29 of the human amylin peptide as defined in SEQ ID NO:3 or amino acids 1-43 of the β-amyloid peptide as defined in SEQ ID NO:1. In all such references, the first number listed is the amino-terminus of this peptide sequence.

β-amyloid peptide naturally occurs as a series of peptides which are 39 to 43 amino acids long, with the shorter, more soluble forms being present in cerebrovascular deposits and the longer forms being found primarily in senile plaques. F. Prelli, et al., Journal of Neurochemistry, 51:648-651 (1988). The primary structure of the 43 amino acid long peptide (β1-43) is depicted in SEQ ID NO:1: 
Asp Ala Glu Phe Arg His Asp Ser Gly Tyr Glu Val His His Gln 15

Lys Leu Val Phe Phe Ala Glu Asp Val Gly Ser Asn Lys Gly Ala 30

Ile Ile Gly Leu Met Val Gly Gly Val Val Ile Ala Thr 43
Even though the full length peptide of SEQ ID NO:1: has sufficient solubility in water for the following experiments, for the purposes of convenience, a more water-soluble form of the peptide is often desired. For that reason, the following examples were performed using peptides containing just the first 40 amino acids of the β-amyloid peptide (β1-40). The sequence of this preferred peptide is SEQ ID NO:2: 
Asp Ala Glu Phe Arg His Asp Ser Gly Tyr Glu Val His His Gln 15

Lys Leu Val Phe Phe Ala Glu Asp Val Gly Ser Asn Lys Gly Ala 30

Ile Ile Gly Leu Met Val Gly Gly Val Val 40
It is understood by those in the art that other fragments of β-amyloid peptide, comprising amino-truncated, carboxy-truncated, or internal deletions, or any combination of these, may be employed in this invention so long as that peptide fragment demonstrates the requisite neurotoxicity.

Amylin is a 37 amino acid peptide having the primary sequence 
 15

 30

 37
hereinafter referred to as SEQ ID NO:3.

The peptide β₂-microglobulin has a molecular weight of about 11,000 Daltons. It can be readily purified from urine samples using known techniques. See, e.g., I. Berggard, et al., Journal of Biological Chemistry, 243:4095 (1968).

### Primary Rat Hippocampal Cell Culture.

Primary hippocampal cultures were prepared essentially as described in P.C. May et al., Neurobiology of Aging, 13:605-607 (1992). High density hippocampal cultures (1.5 x 10⁵ cells/cm²) from E18 rat embryos were cultured in standard 48-well plates coated with 0.5 mg/ml polyethyleneimine. Hippocampal cultures were routinely maintained in Dulbecco's Modified Eagle's Medium (DMEM) containing 4 mM glutamine, 4.5 g/L glucose, 1 mM pyruvate, 50 Units/ml penicillin and 50 µg/ml streptomycin (Pen/Strep) plus 10% fetal bovine serum (GIBCO-BRL, Gaithersburg, Maryland). All experimental treatments were conducted in chemically-defined HEPES-buffered DMEM (minus pyruvate) plus 4 mM Gln, 4.5 g/L glucose and Bottenstein's N-2 supplement (all from GIBCO-BRL).

### Primary Human Cortical Cell Culture

Primary human cortical cultures were prepared essentially as described in P. Seubert, et al., Nature (London) 359:325-327 (1992). Following filtration through Nitrex® nylon screens, cortical tissue was resuspended in a trypsin/EDTA solution [0.05% trypsin plus 0.53 mM EDTA in HBSS (GIBCO-BRL)] and kept at 37°C for 20 minutes. The trypsin was then inactivated by resuspending the cells in neuronal medium [Modified Eagle's Medium (MEM) containing 10% fetal bovine serum, 1% glucose, 1mM sodium pyruvate, 1mM glutamine]. Polyethyleneimine-coated 6.4 mm (96-well) dishes were seeded with 1.0 x 10⁵ cells per well in 0.1 ml neuronal medium. The culture medium was changed twice weekly. Cultures were treated with 10 µM cytosine β-D-arabinofuranoside (Ara-C) in neuronal medium for 3 days on culture days 5-7 and 12-14 to inhibit proliferation of non-neuronal cells.

### Experimental Design.

Amylins were purchased from Bachem, Inc. (Torrance, California), Peninsula Laboratories, Inc. (Belmont, California), Sigma Chemicals (St. Louis, MO) or were synthesized as described infra. Amyloid-β(1-40) [Lot # ZK052] and reverse β-amyloid peptide(40-1) [Lot # ZX299] were purchased from Bachem, Inc. β₂-microglobulin was purchased from Sigma Chemicals (St. Louis, Missouri).

Stock solutions of peptides (1 mM) were freshly prepared in pyrogen-free sterile water and diluted to the indicated concentrations in defined culture media. Rat hippocampal cultures (10-14 days in vitro) were treated with peptides or vehicle for four days. The viability of the rat cortical cultures was visually assessed by phase contrast microscopy and quantified by measuring lactate dehydrogenase (LDH) released into the culture media.

Human cortical cultures (19-22 days in vitro) were treated with peptides or vehicle for three days. Cell viability was visually assessed by phase contrast microscopy and quantified by measuring the reduction of alamarBlue™ (Alamar Biosciences, Inc.) or the tetrazolium salt XTT.

It is understood by those in the art that cell viability, and consequently, toxicity can be measured using other techniques such as monitoring calcium levels. The LDH, XTT and alamarBlue™ techniques as well as the calcium level monitoring assay are described infra.

The data presented reflects results from three to five independent experiments performed in triplicate on different rat hippocampal culture preparations. The human cortical culture data presented reflects results from two independent experiments performed in triplicate.

### Statistical Analysis and Sequence Comparisons.

Main effects of treatment were assessed by Analysis of Variance and, if significant, group means were compared by post-hoc analysis (StatView II®, Abacus Concepts, Berkeley, California). Sequence comparisons were obtained using the GCG™ package (V. 7) developed by the Genetics Computer Group, Inc. (Madison, Wisconsin). Peptide sequences were translated from cDNA sequences and examined for similarities with the BestFit® sub-routine. Percentages reported represent the best region of similarity between the two peptide sequences being compared using standard parameters.

### Peptide Synthesis and Purification

The peptides employed in the instant invention can be prepared using any one of many different protocols or can be purchased from commercial sources. One synthesis protocol employs stepwise solid phase peptide synthesis using a mechanical peptide synthesizer. I. Clark-Lewis, et al., Science, 231:134-139 (1986). This procedure uses commercially available N^{α}-*t*-butoxycarbonyl-protected amino acids and phenylacetamidomethyl or *p*-methylbenzhydrylamine supporting resins. The side chain blocking groups which are commonly used include: O-benzyl groups for aspartate, serine, glutamic acid, and threonine; 2-chlorocarbobenzoxy moieties for blocking lysine; bromocarbobenzoxy groups for tyrosine; tosyl groups for blocking arginine and histidine; and acetamidomethyl for cysteine. No side-chain protection is necessary for asparagine, glutamine, or methionine.

Peptides are deprotected and cleaved from the supporting resin by reaction with liquid hydrogen fluoride (in the presence of anisole) for about 1 hour at 0°C, precipitated in diethyl ether and extracted from the resin with formic acid (about 70%, v/v). To purify and desalt the peptides, crude extracts are chromatographed on size-exclusion columns equilibrated with 70% formic acid.

The peptide content of the eluate is monitored by measurement of its absorption at 280 nm. Fractions within the appropriate molecular weight range of the peptide monomer are pooled and lyophilized. A second purification is done by size-exclusion chromatography in 1 M acetic acid.

An alternative method of purifying the peptides is by the use of reversed-phase high performance liquid chromatography (RP-HPLC). The peptides are loaded onto a nonpolar stationary phase using a solvent such as 0.1% trifluoroacetic acid in water. The peptides are then eluted from the column using a gradient of increasing polarity, such as increasing concentrations of acetonitrile.

The identity and purity of the peptide is then checked using Edman degradation. The Edman degradation may be performed on whole peptides, cyanogen bromide-cleaved peptides, or protease-generated peptides. The Edman degradation may be supplemented with amino acid analysis. Alternatively the identity and purity of the synthesized peptides can be determined using mass spectrometric techniques such as electrospray, laser desorption or fast atom bombardment.

### Neurotoxicity Assay Measuring Calcium Levels

An aliquot of ED 18 cortical cells are seeded into polyethylenimine-coated tissue culture dishes for 3-5 days in vitro before treatment with a 25 µM solution of β-amyloid peptide, either freshly dissolved (predominantly random coil conformation) or aged (7 days, predominantly β-sheet conformation). This neurotoxicity assay is conducted in chemically-defined HEPES-buffered DMEM supplemented with fetal calf serum.

After a two day incubation with the β-amyloid peptide, the elevation of cytosolic calcium (Ca⁺²) concentrations after a glutamate pulse are determined using a fluorescent calcium dye. J. Wahl, et al., Journal of Neurochemistry, 53:1316 (1989). The elevation of intracellular Ca⁺² levels compromises cell integrity.

### Neurotoxicity Assay Measuring XTT

An aliquot of ED 18 cortical cells are seeded into polyethylenimine-coated tissue culture dishes for 3-5 days in vitro before treatment with a 25 µM solution of β-amyloid peptide, either freshly dissolved (predominantly random coil conformation) or aged (7 days, predominantly β-sheet conformation). This assay is conducted in chemically-defined HEPES-buffered DMEM supplemented with fetal calf serum.

These cells are incubated for 3 to 5 days in vitro before treatment with a 25 µM solution of β-amyloid peptide, either freshly dissolved (predominantly random coil conformation) or aged (7 days, predominantly β-sheet conformation). After two days of incubation, cell viability is assessed by measuring the reduction of the tetrazolium salt XTT [2,3-bis(2-methoxy-4-nitro-5-sulfophenyl)-2H-tetrazolium-5-carboxanilide inner salt] as described by N. Roehm, et al., Journal of Immunological Methods, 142:257 (1992).

### Neurotoxicity Assay Measuring LDH

Lactate dehydrogenase (LDH) was measured in 20 µl aliquots of conditioned defined-DMEM using a standard 340 nm kinetic LDH assay (Sigma Catalog Number #228-20) in a 96 well format. Assays were performed at 37°C in a PC-driven EL340 Microplate Biokinetics plate reader (Bio-Tek Instruments) using Delta Soft II software (v. 3.30B, BioMetallics, Inc.) for data analysis. Quality control standards containing normal and elevated levels of serum LDH (for example, Sigma Enzyme Controls 2N and 2E) were run with every assay. Results are expressed as units of LDH/L where 1 unit is defined as the amount of enzyme that will catalyze the formation of 1 micromole of nicotinamide adenine dinucleotide per minute under conditions of the assay.

### Neurotoxicity Assay Measuring alamarBlue™

The alamarBlue™ assay incorporates a proprietary fluorometric/colorimetric growth indicator (Alamar Biosciences, Inc.) based on the detection of metabolic activity. Viable cells cause alamarBlue™ to change from an oxidized (non-fluorescent, blue color) to a reduced (fluorescent, red) form. Neuronal viability as assesed by alamarBlue™ is comparable in sensivity to that obtained by measuring teh release of LDH into the culture media or the reduction of the tetrazolium salt XTT. Assays were performed by replacing the culture media with a 10% alamarBlue™ solution in neuronal medium. Reduction of alamarBlue™ was determined using a Millipore Cytofluor 2350 Scanner (excitation, 560 nm; emission, 590 nm) and CytoCalc™ software (v. 01.00.04, Millipore Corporation). Results are expressed as percent of control (untreated) cultures.

Treatment of mature, high density, rat hippocampal cultures with 50 µM human amylin (Bachem Lot# WI301) resulted in prominent neurodegeneration (Fig 2D.), morphologically similar to that observed in cultures treated with 50 µM β-amyloid peptide (Fig. 2B.). These neurodegenerative changes induced by human amylin were accompanied by a concentration-dependent release of LDH into the culture media (Fig 3, panel A). Human amylin was more potent than β-amyloid peptide with detectable neurotoxicity observed with 10 µM amylin. A similar concentration-response curve was observed with β₂-microglobulin, another amyloidogenic protein. J.M. Campistol and M. Skinner, supra.

Human amylin obtained from 3 independent sources (Bachem, Peninsula, and synthesized in-house as described infra) induced prominent neurodegeneration in cultured rat hippocampal neurons. In contrast, cultures treated with the non-amyloidogenic rat amylin (Bachem Lot # WI357) (Fig. 2C) were indistinguishable from vehicle-treated (H₂O) control wells (Fig. 2A). Consistent with the assessment by phase-contrast microscopy, LDH values in rat amylin-treated cultures were similar to control values (Fig. 3, panel A). Rat amylin obtained from a different source (Peninsula Laboratories) was similarly non-toxic to hippocampal neurons (results not shown).

Only full length human amylin (1-37) was neurotoxic. No toxicity was observed with fragments of human amylin (Fig. 3, panel B) including the ten amino acid peptide containing residues 20-29 important for the secondary structure of amylin. A similar lack of neurotoxicity of the peptide consisting of human amylin(20-29) was recently reported by C.J. Pike, et al., Journal of Neurosciences, 13:1676-1687 (1993), but full length human amylin was apparently not included in that study. These results demonstrate that while the region of amino acids 20-29 is important for determining the final conformation attained by amylin [see, e.g., L.R. McLean and A. Balasubramaniam, Biochimica and Biophysica Acta, 112:317-320 (1992)], it is not of sufficient size to elicit neurotoxicity in mature high density hippocampal cultures.

Treatment of mature, high density, human cortical sultures with 12.5-50 µM human amylin (Bachem lot# WI301) for three days resulted in complete neurodegeneration. (Figure 4). Significant, though not complete, neurodegeneration was also observed in cultures treated with 25-100 µM human β-amyloid peptide (Bachem Lot# ZK600). Human amylin was more potent than β-amyloid peptide with detectable neurotoxicity observed with 3 µM amylin.

A person skilled in the art would understand that, once the assay conditions have been established, a practitioner would then add to the culture media varying amounts of a potential inhibitor of amyloidogenic-peptide neurotoxicity. The decrease in neurotoxicity relative to a control in which no potential inhibitor is added represents an indicator of the effectiveness of the potential inhibitor in decreasing neurotoxicity.

## Claims

1. A method for assaying the effectiveness of agents useful in the treatment or prevention of Alzheimer's disease which comprises:
a. incubating potential inhibitors of neurotoxicity with an amyloidogenic amylin;
b. measuring the neurotoxic properties of each amylin/potential inhibitor mixture; and
c. detecting reduction in the neurotoxicity relative to a control.

2. A method as claimed in Claim 1 wherein said amyloidogenic amylin is human amylin.

3. A method for assaying the effectiveness of agents useful in the treatment or prevention of Alzheimer's disease which comprises:
a. incubating potential inhibitors of neurotoxicity with an amyloidogenic β₂-microglobulin;
b. measuring the neurotoxic properties of each amyloidogenic β₂-microglobulin/potential inhibitor mixture; and
c. detecting reduction in the neurotoxicity relative to a control.

4. A method for assessing the effectiveness of an agent useful for ameliorating the neurotoxic effects of a condition associated with accumulation of β-amyloid peptide which comprises
a. incubating potential inhibitors of neurotoxicity with an amyloidogenic amylin;
b. measuring the neurotoxic properties of each amylin/potential inhibitor mixture; and
c. detecting reduction in the neurotoxicity relative to a control.

5. A method as claimed in Claim 4 wherein the condition associated with accumulation of β-amyloid peptide is Alzheimer's disease or Down's Syndrome.

6. A method for assessing the effectiveness of an agent useful for ameliorating the neurotoxic effects of a condition associated with accumulation of β-amyloid peptide which comprises
a. incubating potential inhibitors of neurotoxicity with an amyloidogenic β₂-microglobulin;
b. measuring the neurotoxic properties of each amylin/potential inhibitor mixture; and
c. detecting reduction in the neurotoxicity relative to a control in which no poteintial inhibitor is added.
